# FASCICULE DE BREVET EUROPEEN

(11) **EP 1 222 902 B1**
(45) Date de publication et mention de la délivrance du brevet: **04.08.2004**
(21) Numéro de dépôt: 01402543.1
(22) Date de dépôt: 02.10.2001
(51) Int. Cl.: A61F 2/16

(54) **Lentilles intraoculaires**
Intraokuläre Linsen
Intraocular lenses

(30) Priorité: 02.10.2000 FR 0012531
(43) Date de publication de la demande: 17.07.2002
(73) Titulaire: Biotech, 45300 Pithivers (FR)
(72) Inventeur: Liffredo, Renato, 20015 Parabiago (IT); Delage, Denis, 45300 Yevre le Chatel (FR)
(74) Mandataire: Debay, Yves

(56) Documents cités:
- FR-A- 2 765 797
- FR-A- 2 766 699
- US-A- 4 657 546

## Description

La présente invention concerne une lentille intraoculaire destinée à être insérée dans le sac capsulaire de l'oeil.

Les lentilles intraoculaires sont destinées à la correction de l'aphakie lors de l'opération de la cataracte. La cataracte est caractérisée par une perte progressive de la vision par opacification du cristallin chez le patient. Par une opération chirurgicale, le cristallin opacifié pourra être extrait, et remplacé par un cristallin artificiel nommé lentille intraoculaire. La lentille intraoculaire peut être placée dans la chambre antérieure, devant l'iris, ou dans la chambre postérieure, derrière l'iris, en appui ciliaire ou dans le sac capsulaire du cristallin. La lentille est composée de deux parties, une partie optique assurant la vision qui peut-être monofocale ou multifocale, et une partie de maintien ou haptique (anses en français), qui agira en interaction avec les tissus, par poussée mécanique et croissance cellulaire et qui assurera la position de la lentille dans l'oeil. Aujourd'hui, pour remplacer le cristallin naturel, il existe deux types d'implants, l'implant dit rigide ou l'implant dit souple.

Le matériau utilisé pour les lentilles intraoculaires rigides est en général le polyméthacrylate de méthyle (PMMA). L'implant souple peut se plier et être introduit par une très faible incision cornéenne ou sclérale d'environ 3 millimètres, après extraction du noyau cristallin du sac capsulaire. Cette technique permet de diminuer l'astigmatisme résiduel. De nombreuses lentilles intraoculaires en matière souple ont déjà été proposées. De telles lentilles sont réalisées par exemple en polysiloxanes, ou en polymères acryliques hydrophobes souples ou hydrophiles (copolyHEMA). Les lentilles intraoculaires en co-poly-hema sont obtenues par usinage à l'état sec, le matériau sera ensuite hydraté pour le rendre souple.

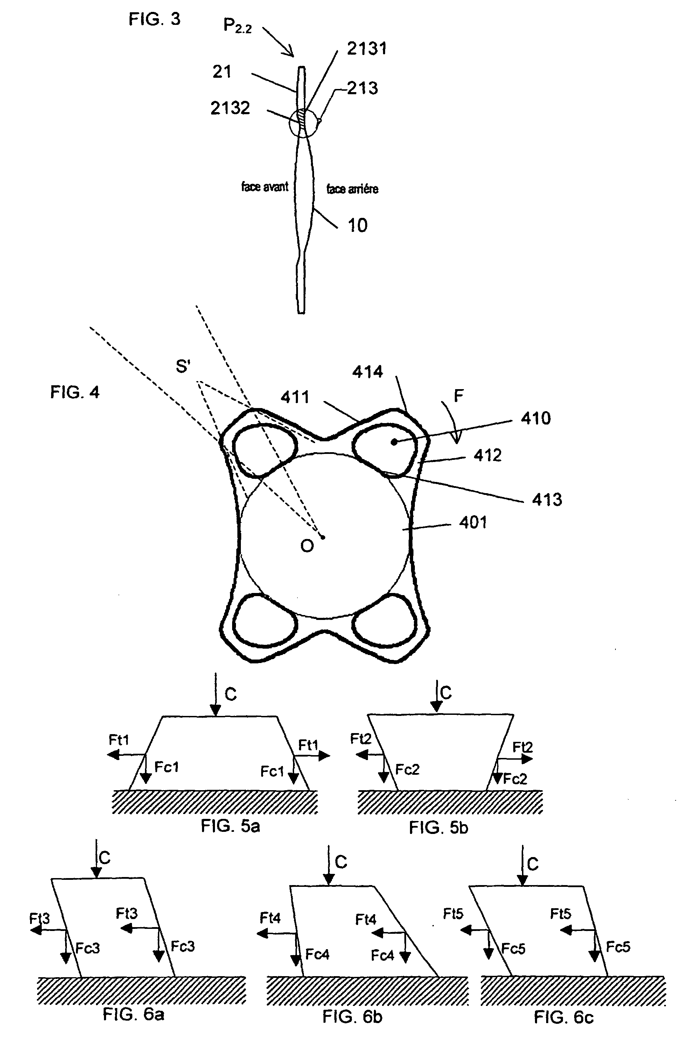

Il est connu également dans l'art antérieur, par la demande de brevet FR 2 766 699 une lentille intraoculaire monobloc souple ayant pour but d'éviter un déplacement de l'optique de la lentille selon l'axe optique lorsque la lentille est mise en place. Cette lentille est représentée à la figure 4. Afin d'atteindre ce but, ce document propose d'utiliser des anses (410) fermées dont les jambes (411, 412) ne sont pas radiales. Ainsi, en théorie, les efforts transmis par les jambes (411, 412) sur la partie (401) optique lorsque la lentille est en place dans le sac capsulaire de l'oeil ne comprennent pas de composante radiale. Par conséquent, la partie optique (401) ne devrait pas se déplacer ou se déformer. Cependant, il faut noter que selon la figure 4, pour chaque anse (410), les axes longitudinaux des jambes (411, 412) sont sécants en un point S' qui est situé dans le secteur angulaire de centre O et d'arc défini par la portion de contact (414) de l'anse joignant les extrémités libres des jambes (411, 412). Une fois la lentille en place dans le cristallin, des forces centripètes vont s'appliquer sur chaque anse (410). Compte tenu de la géométrie de chaque anse définie précédemment, ces forces vont induire sur chaque jambe (411, 412) un moment de rotation. Selon la figure 4, ces deux moments de rotation seront de signe contraire. Si ces deux moments ont la même valeur alors l'anse va subir une simple compression, ce qui entraîne nécessairement un flambage des jambes. Si la somme des moments n'est pas nulle, il y a à la fois compression de la jambe ayant le moment le plus faible et pivotement de l'anse dans la direction du moment le plus fort. Ainsi, quel que soit le cas de figure au moins une jambe subit une compression. Ce phénomène de compression est également illustré sur la figure 5a sur laquelle est représentée de manière schématique la forme d'une anse telle que celle utilisée dans l'art antérieur représenté en figure 4. Sur cette figure, la compression (C) qui s'exerce sur l'anse lorsque la lentille est en place, engendre sur chaque jambe (411, 412) l'exercice d'une composante centripète (Fc1) et d'une composante tangentielle (Ft1). Du fait de la géométrie particulière de l'anse de la lentille, ces composantes tangentielles (Ft1) sont de signe contraire et dirigées dans des sens opposées. En fonction de la valeur de ces forces, les phénomènes décrits ci-dessus vont s'appliquer sur chaque jambe (411, 412). Ces phénomènes vont générer une accumulation de contraintes qui va se traduire, soit par une déformation aléatoire de la ou des jambes entraînant une instabilité dans la position de la partie optique, soit par une pression supplémentaire exercée sur les tissus périphériques du sac capsulaire pouvant entraîner des complications pour le patient. Ces phénomènes pourront se produire également lorsque chaque anse aura une géométrie avec ses deux jambes non pas orientées vers l'extérieur mais vers l'intérieur comme représenté en figure 5b. Dans ce cas, les composantes tangentielles (Ft2) seront dirigées l'une vers l'autre.

Il est connu dans l'art antérieur, par le document FR 2 765 797, un implant intraoculaire monobloc souple comprenant deux ensembles haptiques formés chacun par deux bandes haptiques qui sont solidaires de la partie optique à une extrémité et par une paire de portions de maintien à l'autre extrémité, les portions de maintien étant reliées entre elles par un élément de liaison. Cet implant intraoculaire ne permet pas de supprimer les phénomènes de contrainte susmentionnés.

La présente invention a donc pour objet de pallier les inconvénients de l'art antérieur en proposant une lentille intraoculaire dont les parties haptiques n'entraînent ni déplacement de la partie optique suivant l'axe optique, ni accumulation de contrainte sur la partie optique ou sur les tissus périphériques.

Cet objectif est atteint par une lentille intraoculaire selon les revendications 1 et 2.

Des développements supplémentaires de l'invention sont décrits dans les revendications 3 à 10.

L'invention, avec ses caractéristiques et avantages, ressortira plus clairement à la lecture de la description faite en référence aux dessins annexés dans lesquels :
- la figure 1 représente une vue de face de la lentille intraoculaire selon l'invention avant insertion dans le sac capsulaire,
- la figure 2 représente une vue de face de la lentille intraoculaire selon l'invention sous contrainte après insertion dans le sac capsulaire,
- la figure 3 représente une vue de coté de la lentille intraoculaire selon l'invention,
- la figure 4 représente une lentille intraoculaire monobloc souple selon l'art antérieur,
- les figures 5a à 6c représentent de manière schématique les efforts de compression s'appliquant sur une anse lorsque la lentille est en place dans le sac capsulaire, pour différentes géométries d'anse.

Avant de décrire l'invention, le contexte technique et les problèmes rencontrés avec les lentilles connues vont être rappelés. Les lentilles intraoculaires souples qui n'ont pas adopté la forme anse ouverte avaient une forme dite navette, particulièrement adaptée à l'introduction par injecteur. Ce modèle se déforme par flambement, projetant l'optique vers la capsule arrière, ou par déformation locale en extrémité.

Par la suite, les designs se sont déclinés sous forme quadripode ou tripode avec des anses ajourées ou non.

Dans ces configurations, le phénomène de flambement est accru ainsi que les effets indésirables associés, notamment le décentrage de la lentille, l'augmentation du risque de cataracte secondaire généré par un mauvais contact lentille/capsule postérieure, l'erreur de réfraction par défocalisation en cas de non-projection postérieure, la diminution de la sensibilité au contraste (perte de résolution) par déformation astigmate de l'optique. Selon l'art antérieur connu, il existe des formes d'anse dont les jambages sont radiaux. Dans ce cas, les anses se déformeront hors du plan de la lentille. Une autre solution est donnée par la lentille décrite dans le document FR 2 766 699 commenté précédemment en référence à la figure 4 et 5a. Cependant pour ces lentilles, lors de la projection, la transmission d'effort des anses à l'optique peut s'accompagner d'une déformation astigmate de l'optique. De même, la lentille, en position déformée, offre une très faible résistance aux efforts radiaux, une faible pression suffit à augmenter la projection, accroît le phénomène d'instabilité et diminue les capacités d'auto-centrage. En cas de rétraction du sac, les phénomènes de projection sont accentués.

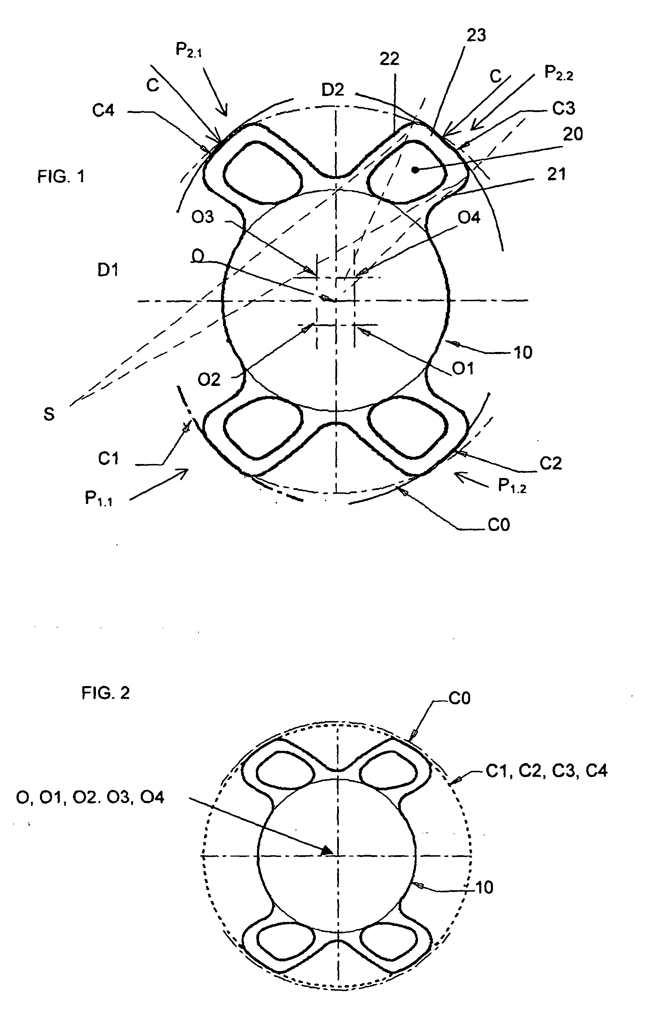

L'invention va à présent être décrite en référence aux figures 1 à 3. A titre d'exemple, la lentille représentée aux figures 1 à 3 est du type lentille souple monobloc.

De façon connue, la lentille intraoculaire comprend une partie optique (10) sensiblement circulaire et des parties (P_{1.1}, P_{1.2},P_{2.1}, P_{2.2}) de maintien ou haptiques solidaires de la périphérie de la partie optique (10). Les parties haptiques comprennent deux paires d'anses (P_{1.1}, P_{1.2} respectivement P_{2.1}, P_{2.2}), chaque paire étant symétrique par rapport à un premier diamètre (D1) de la partie optique (10). De même, la première anse d'une paire d'anses est symétrique à la deuxième anse de la même paire par rapport à un deuxième diamètre (D2) de la partie optique (10). Selon l'invention, le premier (D1) et le deuxième diamètre (D2) sont perpendiculaires. Chaque anse (P_{1.1}, P_{1.2}, P_{2.1}, P_{2.2}) comprend un premier (21) et un deuxième (22) bras ou jambage dont une première extrémité est solidaire de la périphérie de la partie (10) optique.

Selon une première variante de l'invention, les axes longitudinaux des jambages (21, 22) de chaque anse (20) sont sécants en un point (S) et ne sont pas radiaux par rapport au centre optique (O) de la lentille. Le point (S) d'intersection entre les deux jambages (21, 22) de chaque anse (20) est située en dehors du secteur angulaire dont le centre est confondu avec le centre (O) optique de la partie optique (10) et dont l'arc est constitué par la portion (23) de contact joignant les deuxièmes extrémités des jambages (21, 22) d'une même anse (20). Il faut comprendre que le secteur angulaire, tel que défini, s'étend à la fois en direction de l'anse (20) dont la portion (23) de contact, sert d'arc au secteur angulaire, mais également dans la direction opposée. Cet aspect peut se traduire également par le fait que pour chaque anse (P_{1.1}, P_{1.2}, P_{2.1}, P_{2.2}), l'axe longitudinal d'un jambage (21, 22) de cette anse (20) est inclinée du même côté par rapport à sa radiale que l'axe longitudinal de l'autre jambage de l'anse par rapport à sa radiale, la radiale d'un jambage étant définie par l'axe passant par la deuxième extrémité de chaque jambage (21, 22) et le centre optique (O).

Ces configurations particulières perment d'obtenir une flexion des anses (P_{1.1}, P_{1.2}, P_{2.1}, P_{2.2}) dans le plan de la partie optique (10), sans provoquer d'accumulation de contrainte. En effet, lorsque la lentille est en place dans le sac capsulaire, les anses vont venir en appui sur les tissus intérieurs du sac capsulaire et subir alors des contraintes mécaniques de compression. Sous l'action de ces contraintes et compte tenu de la configuration des jambages (21, 22), un moment de rotation de même sens va s'appliquer sur chaque jambage de chaque anse de sorte que les jambages d'une même anse vont pivoter dans le même sens et provoquer la flexion de l'anse dans le plan de la partie (10) optique. De cette façon toutes les contraintes mécaniques vont être libérées dans la flexion des anses. Ainsi, les efforts des anses seront appliqués uniformément sur les tissus et assureront un maintien de la lentille dans la position voulue. De même, la flexion des anses entraîne un minimum de déformations mécaniques à la partie optique (10). Ceci est illustré par les figures 6a à 6c sur lesquelles on peut voir schématiquement plusieurs géométries d'anses différentes. Sur ces figures, les axes longitudinaux des jambages sont inclinés du même côté par rapport à leur radiale respective. La compression (C) agissant sur chaque anse (P_{1.1}, P_{1.2}, P_{2.1}, P_{2.2}) lorsque la lentille est en place génère sur chaque jambage (21, 22) d'une anse (20), comme dans l'art antérieur, une composante centripète (Fc3, Fc4, Fc5) et une composante tangentielle (Ft3, Ft4, Ft5). Toutefois, selon l'invention, du fait de la géométrie particulière des anses, les composantes tangentielles (Ft3, Ft4, Ft5) sont orientées dans le même sens ce qui provoquera la flexion de l'anse dans le plan de la partie (10) optique

Plus l'inclinaison entre les anses et les rayons de la partie optique (10) est importante, plus l'amplitude de flexion est grande mais plus la résistance des jambages est faible. Une forte amplitude permet de compenser des variations importantes de diamètre entre la position de repos où la lentille est hors du sac capsulaire et une position de travail où la lentille est insérée dans le sac capsulaire.

Selon une autre variante de réalisation, les axes longitudinaux des jambages (21, 22) de chaque anse sont parallèles. Dans ce cas, l'axe passant par le centre optique et parallèle aux axes des jambages (21, 22), correspondant autrement dit à l'axe des jambages reporté au centre optique O, doit être situé à l'extérieur du secteur angulaire de centre O et d'arc correspondant à la portion (23) de contact.

Il faut noter que cette définition peut s'appliquer à la variante de réalisation dans laquelle les axes longitudinaux des jambages (21, 22) sont sécants. En effet, pour que le point (S) d'intersection entre les deux jambages (21, 22) de chaque anse (20) soit situé en dehors du secteur angulaire défini précédemment il faut que les axes longitudinaux des jambages (21, 22) reportés au centre optique (O) ne soient pas compris dans le secteur angulaire.

Comme décrit précédemment, ceci peut se traduire géométriquement par le fait que les axes longitudinaux des jambages (21, 22) d'une anse (20) doivent être inclinés du même côté par rapport à leur radiale respective, la radiale étant définie comme décrit ci-dessus.

Les deuxièmes extrémités des jambages (21, 22) de chaque anse sont reliées par une portion (23) de maintien ou portée qui assure le contact entre les parties haptiques (P_{1.1}, P_{1.2}, P_{2.1}, P22) et les tissus du sac capsulaire après introduction de la lentille. Selon l'invention, chaque portée (23) est inscrite dans un cercle (C1 à C4) de centre (O1 à O4) distinct du centre (O) optique. Les centres des cercles (C1 à C4), dit d'excentricité, dans lesquels sont inscrites les portées sont situés dans la partie optique. Dans une variante de réalisation, les centres (O1 à O4) des cercles (C1 à C4) sont symétriques deux à deux, soit par rapport au premier diamètre (D1), soit par rapport au deuxième diamètre (D2), soit par rapport au centre (O) optique. Cette caractéristique permet d'obtenir une meilleure stabilité latérale de la lentille, ainsi qu'un meilleur auto-centrage.

Lors de la flexion des anses pendant l'introduction de la lentille, les centres (O1 à O4) des cercles (C1 à C4) d'excentricité vont se déplacer jusqu'à ce qu'ils soient confondus ou tout au moins les plus proches possible du centre (O) optique. En d'autres termes, à l'issue de la mise en place de la lentille selon l'invention, les cercles (C1 à C4) sont sensiblement confondus avec un cercle (C0) définissant les limites du sac capsulaire.

De même, la configuration des jambages (21, 22) des anses telles que définies précédemment, induit une différence de longueur des jambages (21, 22). Par conséquent, lors de la flexion, les efforts dus à la flexion seront plus importants sur le jambage (21) le plus court. En utilisant une portée excentrée par rapport au centre optique, l'effort de flexion est équilibré sur les deux jambages (21, 22). L'excentration de la portée (23) est fonction de l'inclinaison de l'anse. Plus l'inclinaison (I) des anses est importante, plus l'excentration, c'est-à-dire la distance entre le centre (O) optique et le centre du cercle (C2) tangent à la portée (23), est importante, plus les efforts seront reportés sur le jambage (22) le plus long.

Afin de compenser la résistance en flexion du jambage le plus court en augmentant sa résistance, la surface de la section du jambage le plus court (21) est plus important que la surface de la section du jambage le plus long (22).

Selon une variante de réalisation, la section des jambages est de forme sensiblement rectangulaire avec les angles arrondis.

Lors de l'introduction de la lentille dont les anses (P_{1.1}, P_{1.2}, P_{2.1}, P_{2.2}) ont des portées excentrées, la flexion des anses et donc des jambages (21, 22), entraîne un centrage des cercles (C1 à C4) sur lesquels sont inscrites les portées sur le cercle (C0) de centre optique (O). Ainsi, après l'introduction de la lentille dans le sac capsulaire, toute la longueur de chaque portée (23) est en contact étroit avec les tissus périphériques, ce qui entraîne une répartition des efforts et donc moins de risque de complication postopératoire.

La figure 3 représente une variante de réalisation de l'invention. Selon cette variante, la jonction (213) entre la première extrémité de chaque jambage (21, 22) et la périphérie de la partie optique (10) est de forme déterminée pour empêcher une projection de la lentille vers l'avant lors de son introduction dans le sac capsulaire. Par projection, il faut comprendre déplacement de la partie optique selon l'axe optique et inclination des anses par rapport au plan de la partie optique (10). Selon l'invention, la section transversale ou profil de la jonction (213) est dissymétrique par rapport à un plan perpendiculaire à l'axe optique de la lentille. Ainsi, selon l'invention, la face (2131) de la jonction (213) située dans le prolongement de la face avant de la partie (10) optique est sensiblement perpendiculaire à l'axe optique. La face (2132) de la jonction (213) située dans le prolongement de la face arrière de la partie (10) optique forme un angle déterminé avec la face (2131) de la jonction (213) située dans le prolongement de la face avant de sorte que la partie de la jonction (213) adjacente à la partie (10) optique est plus étroite que la partie de la jonction (213) opposée à la partie (10) optique. Ainsi, on comprend que compte tenu de cet angle, la projection de la partie (10) optique vers l'arrière est privilégiée par rapport à la projection vers l'avant.

Selon une autre variante, La projection arrière peut aussi être amplifiée en utilisant un plan d'anse incliné par rapport à l'optique.

Les figures 1 à 4 représentent une lentille monobloc souple. Cependant le principe de l'invention qui vient d'être décrit est transposable aux lentilles intraoculaires rigides. Ainsi, pour une lentille intraoculaire rigide, les anses seront rapportées sur la périphérie de la lentille sur des emplacements prévus à cet effet.

Ainsi, la lentille intraoculaire selon l'invention se caractérise en ce que chaque anse (P1.1, P1.2, P2.1, P2.2) comprend deux bras (21, 22) ou jambages dont une première extrémité est solidaire sur la périphérie de la partie optique (10) et, pour chaque anse (P1.1, P1.2, P2.1, P2.2), les axes longitudinaux des jambages (21, 22) sont sécants en un point (S) n'appartenant pas à un secteur angulaire dont le centre est confondu avec le centre (O) optique de la partie optique (10) et dont l'arc est constitué par une portion (23) de contact joignant les deuxièmes extrémités des jambages d'une même anse.

Dans un autre mode de réalisation, chaque anse (P1.1, P1.2, P2.1, P2.2) comprend deux bras (21, 22) ou jambages dont une première extrémité est solidaire sur la périphérie de la partie optique (10) et, pour chaque anse (P1.1, P1.2, P2.1, P2.2), les axes longitudinaux des jambages (21, 22) sont parallèle, et les axe longitudinaux des jambages (21, 22) ne sont pas compris dans un secteur angulaire dont le centre est confondu avec le centre (O) optique de la partie optique (10) et dont l'arc est constitué par une portion (23) de contact joignant les deuxièmes extrémités des jambages d'une même anse.

Dans un autre mode de réalisation, les deuxièmes extrémités des jambages (21, 22) de chaque anse sont reliées par une portion (23) de contact ou portée inscrite ou tangente à un cercle (C1 à C4) non concentrique avec le centre optique (O) de la partie (10) optique.

Dans un autre mode de réalisation, pour chaque anse, la surface de la section du jambage (21) le plus court est plus importante que la surface de la section du jambage (22) le plus long.

Dans un autre mode de réalisation, les sections transversales des jambages (21, 22) sont de forme rectangulaire avec les angles arrondis.

Dans un autre mode de réalisation, la jonction (213) entre chaque jambage (21, 22) de chaque anse (P1.1, P1.2, P2.1, P2.2) possède un profil longitudinal dissymétrique.

Dans un autre mode de réalisation, la jonction (213) entre chaque jambage (21, 22) de chaque anse (P1.1, P1.2, P2.1, P2.2) est comprise dans un plan formant un angle déterminé avec le plan de la partie (10) optique

Dans un autre mode de réalisation, la face arrière (2131) de la jonction est comprise dans un plan perpendiculaire à l'axe optique et la face avant (2132) de la jonction (213) est comprise dans un plan formant un angle aigu avec l'axe optique de sorte qu'une projection vers l'arrière de la partie optique soit privilégiée.

Dans un autre mode de réalisation, la lentille intraoculaire est monobloc et est réalisée dans un matériau souple.

Dans un autre mode de réalisation, la lentille intraoculaire est de type rigide, la partie optique étant rigide et les anses (P1.1, P1.2, P2.1, P2.2) étant souples et rapportées ou usinées sur la périphérie de la partie optique.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration mais peuvent être modifiés dans le domaine défini par la portée des revendications jointes.

## Revendications

1. Lentille intraoculaire comprenant une partie optique (10) sensible en forme de disque et une partie de maintien ou partie haptique comprenant deux paires d'anses (P_{1.1}, P_{1.2} P_{2.1}, P_{2.2}) fermées disposées symétriquement par rapport à un premier diamètre (D1) de la partie optique (10), les anses (P_{1.1}, P_{1.2} P_{2.1}, P_{2.2}) de chaque paire étant symétriques entre elles par rapport à un deuxième diamètre (D2) sensiblement perpendiculaire au premier diamètre (D1), **caractérisée en ce que** chaque anse (P_{1.1}, P_{1.2}, P_{2.1}, P_{2.2}) comprend deux bras (21, 22) ou jambages ayant chacun une première extrémité solidaire sur la périphérie de la partie optique (10) et une seconde extrémité solidaire d'une portion (23) de contact joignant les deux jambages (21, 22) et, pour chaque anse (P_{1.1}, P_{1.2}, P_{2.1}, P_{2.2}), les axes longitudinaux des jambages (21, 22) sont sécants en un point (S) n'appartenant pas à un secteur angulaire dont le centre est confondu avec le centre (O) optique de la partie optique (10) et dont l'arc est constitué par ladite portion (23) de contact joignant les deuxièmes extrémités des jambages d'une même anse.

2. Lentille intraoculaire comprenant une partie optique (10) sensible en forme de disque et une partie de maintien ou partie haptique comprenant deux paires d'anses (P_{1.1}, P_{1.2} P_{2.1}, P_{2.2}) fermées disposées symétriquement par rapport à un premier diamètre (D1) de la partie optique (10), les anses (P_{1.1}, P_{1.2} P_{2.1}, P_{2.2}) de chaque paire étant symétriques entre elles par rapport à un deuxième diamètre (D2) sensiblement perpendiculaire au premier diamètre (D1), **caractérisée en ce que** chaque anse (P_{1.1}, P_{1.2}, P_{2.1}, P_{2.2}) comprend deux bras (21, 22) ou jambages dont une première extrémité est solidaire sur la périphérie de la partie optique (10) et, pour chaque anse (P_{1.1}, P_{1.2}, P_{2.1}, P_{2.2}), les axes longitudinaux des jambages (21, 22) sont parallèles entre eux, et les axes longitudinaux des jambages (21, 22) reportés au centre optique ne sont pas compris dans un secteur angulaire dont le centre est confondu avec le centre (O) optique de la partie optique (10) et dont l'arc est constitué par une portion (23) de contact joignant les deuxièmes extrémités des jambages d'une même anse.

3. Lentille intraoculaire selon la revendication 1 ou 2, **caractérisée en ce que** chaque portion (23) de contact ou portée est inscrite ou tangente à un cercle (C1 à C4) non concentrique avec le centre optique (O) de la partie (10) optique.

4. Lentille intraoculaire selon l'une des revendications 1 à 3, **caractérisée en ce que** pour chaque anse, la surface de la section du jambage (21) le plus court est plus importante que la surface de la section du jambage (22) le plus long.

5. Lentille intraoculaire selon la revendication 4, **caractérisée en ce que** les sections transversales des jambages (21, 22) sont de forme rectangulaire avec les angles arrondis.

6. Lentille intraoculaire selon l'une des revendications 1 à 5, **caractérisée en ce que** la jonction (213) entre chaque jambage (21, 22) de chaque anse (P_{1.1}, P_{1.2}, P_{2.1}, P_{2.2}) possède un profil longitudinal dissymétrique.

7. Lentille intraoculaire selon l'une des revendications 1 à 6, **caractérisée en ce que** la jonction (213) entre chaque jambage (21, 22) de chaque anse (P1.1, P1.2, P2.1, P2.2) est comprise dans un plan formant un angle déterminé avec le plan de la partie (10) optique.

8. Lentille intraoculaire selon l'une des revendications 1 à 7, **caractérisée en ce que** la face arrière (2131) de la jonction est comprise dans un plan perpendiculaire à l'axe optique et la face avant (2132) de la jonction (213) est comprise dans un plan formant un angle aigu avec l'axe optique de sorte qu'une projection vers l'arrière de la partie optique soit privilégiée.

9. Lentille intraoculaire selon l'une des revendications 1 à 8, **caractérisée en ce que** la lentille intraoculaire est monobloc et est réalisée dans un matériau souple.

10. Lentille intraoculaire selon l'une des revendications 1 à 8, **caractérisée en ce que** la lentille intraoculaire est de type rigide, la partie optique étant rigide et les anses (P_{1.1}, P_{1.2}, P_{2.1}, P_{2.2}) étant souples et rapportées ou usinées sur la périphérie de la partie optique.

## Patentansprüche

1. Intraokuläre Linse mit
einem optischen Abschnitt (10) im Wesentlichen in Form einer Scheibe und
einem Halteabschnitt oder haptischen Abschnitt mit zwei Paaren von geschlossenen Henkeln (P_{1.1}, P_{1.2}, P_{2.1}, P_{2.2}), die symmetrisch mit Bezug auf einen ersten Durchmesser (D1) des optischen Abschnitts (10) angeordnet sind,
wobei die Henkel (P_{1.1}, P_{1.2}, P_{2.1}, P_{2.2}) jedes Paares symmetrisch zueinander mit Bezug auf einen zweiten Durchmesser (D2) sind, der im Wesentlichen senkrecht zu dem ersten Durchmesser (D1) ist,
**dadurch gekennzeichnet, dass**
jeder Henkel (P_{1.1}, P_{1.2}, P_{2.1}, P_{2.2}) zwei Arme oder Schenkel (21, 22) enthält, von denen jeder ein erstes Ende aufweist, das mit einem Rand des optischen Abschnitts (10) verbunden ist, und ein zweites Ende, das mit einem Kontaktabschnitt (23) verbunden ist, der die zwei Schenkel (21, 22) verbindet, und
für jeden Henkel (P_{1.1}, P_{1.2}, P_{2.1}, P_{2.2}) die Längsachsen der Schenkel (21, 22) sich in einem Punkt (S) schneiden, der nicht zu einem Winkelsektor gehört, dessen Mittelpunkt mit dem optischen Zentrum (O) des optischen Abschnitts (10) zusammenfällt und dessen Bogen durch den Kontaktabschnitt (23) gebildet wird, der die zweiten Enden der Schenkel desselben Henkels verbindet.

2. Intraokuläre Linse mit
einem optischen Abschnitt (10) im Wesentlichen in Form einer Scheibe und
einem Halteabschnitt oder haptischen Abschnitt mit zwei Paaren von geschlossenen Henkeln (P_{1.1}, P_{1.2}, P_{2.1}, P_{2.2}), die symmetrisch mit Bezug auf einen ersten Durchmesser (D1) des optischen Abschnitts (10) angeordnet sind,
wobei die Henkel (P_{1.1}, P_{1.2}, P_{2.1}, P_{2.2}) jedes Paares symmetrisch zueinander mit Bezug auf einen zweiten Durchmesser (D2) sind, der im Wesentlichen senkrecht zu dem ersten Durchmesser (D1) ist,
**dadurch gekennzeichnet, dass**
jeder Henkel (P_{1.1}, P_{1.2}, P_{2.1}, P_{2.2}) zwei Arme oder Schenkel (21, 22) enthält, deren erstes Ende mit einem Rand des optischen Abschnitts (10) verbunden ist,
für jeden Henkel (P_{1.1}, P_{1.2}, P_{2.1}, P_{2.2}) die Längsachsen der Schenkel (21, 22) zueinander parallel sind, und
die in das optische Zentrum versetzten Längsachsen der Schenkel (21, 22) nicht in einem Winkelsektor enthalten sind, dessen Mittelpunkt mit dem optischen Zentrum (O) des optischen Abschnitts (10) zusammenfällt und dessen Bogen durch einen Kontaktabschnitt (23) gebildet wird, der die zweiten Enden der Schenkel desselben Henkels verbindet.

3. Intraokuläre Linse nach Anspruch 1 oder 2, **dadurch gekennzeichnet, dass** jeder Kontaktabschnitt oder Träger (23) eingeschrieben in oder tangential zu einem Kreis (C1-C4) ist, der nicht mit dem optischen Zentrum (O) des optischen Abschnitts (10) konzentrisch ist.

4. Intraokuläre Linse nach einem der Ansprüche 1 bis 3, **dadurch gekennzeichnet, dass** für jeden Henkel die Schnittfläche des kürzesten Schenkels (21) größer ist als die Schnittfläche des längsten Schenkels (22).

5. Intraokuläre Linse nach Anspruch 4, **dadurch gekennzeichnet, dass** die Querschnitte der Schenkel (21, 22) rechteckförmig mit abgerundeten Kanten sind.

6. Intraokuläre Linse nach einem der Ansprüche 1 bis 5, **dadurch gekennzeichnet, dass** die Verbindungsstelle (213) zwischen jedem Schenkel (21, 22) jedes Henkels (P_{1.1}, P_{1.2}, P_{2.1}, P_{2.2}) ein unsymmetrisches Längsprofil aufweist.

7. Intraokuläre Linse nach einem der Ansprüche 1 bis 6, **dadurch gekennzeichnet, dass** die Verbindungsstelle (213) zwischen jedem Schenkel (21, 22) jedes Henkels (P_{1.1}, P_{1.2}, P_{2.1}, P_{2.2}) in einer Ebene enthalten ist, die einen vorbestimmten Winkel mit der Ebene des optischen Abschnitts (10) bildet.

8. Intraokuläre Linse nach einem der Ansprüche 1 bis 7, **dadurch gekennzeichnet, dass**
die Rückfläche (2131) der Verbindungsstelle in einer Ebene senkrecht zu der optischen Achse enthalten ist und
die Vorderfläche (2132) der Verbindungsstelle (213) in einer Ebene enthalten ist, die einen spitzen Winkel mit der optischen Achse bildet, so dass ein Vorspringen des optischen Abschnitt nach hinten begünstigt ist.

9. Intraokuläre Linse nach einem der Ansprüche 1 bis 8, **dadurch gekennzeichnet, dass** die intraokuläre Linse einstückig und aus einem biegsamen Material ausgebildet ist.

10. Intraokuläre Linse nach einem der Ansprüche 1 bis 8, **dadurch gekennzeichnet, dass**
die intraokuläre Linse von einem starren Typ ist,
wobei der optische Abschnitt starr ist und
die Henkel (P_{1.1}, P_{1.2}, P_{2.1}, P_{2.2}) biegbar und an dem Rand des optischen Abschnitts angestückt oder angearbeitet sind.

## Claims

1. Intraocular lens comprising a sensitive disc-shaped optical part (10) and a support part or haptic part comprising two pairs of closed loops (P_{1.1}, P_{1.2}, P_{2.1}, P_{2.2}) arranged symmetrically with respect to a first diameter (D1) of the optical part (10), the loops (P_{1.1}, P_{1.2}, P_{2.1}, P_{2.2}) in each pair being symmetrical with each other with respect to a second diameter (D2) substantially perpendicular to the first diameter (D1), **characterised in that** each loop (P_{1.1}, P_{1.2}, P_{2.1}, P_{2.2}) comprises two arms (21, 22) or legs each having a first end integral with the periphery of the optical part (10) and a second end integral with a contact portion (23) joining the two legs (21, 22) and, for each loop (P_{1.1}, P_{1.2}, P_{2.1}, P_{2.2}), the longitudinal axes of the legs (21, 22) intersect at a point (S) that does not belong to an angular sector the centre of which is the same as the optical centre (O) of the optical part (10) and the arc of which is constituted by said contact portion (23) joining the second ends of the legs of the same loop.

2. Intraocular lens comprising a sensitive disc-shaped optical part (10) and a support part or haptic part comprising two pairs of closed loops (P_{1.1}, P_{1.2}, P_{2.1}, P_{2.2}) arranged symmetrically with respect to a first diameter (D1) of the optical part (10), the loops (P_{1.1}, P_{1.2}, P_{2.1}, P_{2.2}) in each pair being symmetrical with each other with respect to a second diameter (D2) substantially perpendicular to the first diameter (D1), **characterised in that** each loop (P_{1.1}, P_{1.2}, P_{2.1}, P_{2.2}) comprises two arms (21, 22) or legs in which a first end is integral with the periphery of the optical part (10) and, for each loop (P_{1.1}, P_{1.2}, P_{2.1}, P_{2.2}), the longitudinal axes of the legs (21, 22) are parallel to each other, and the longitudinal axes of the legs (21, 22) relative to the optical centre do not lie within an angular sector the centre of which is the same as the optical centre (O) of the optical part (10) and the arc of which is constituted by a contact portion (23) joining the second ends of the legs of the same loop.

3. Intraocular lens according to Claim 1 or 2, **characterised in that** each contact portion (23) or contact surface is inscribed within or tangent to a circle (C1) to (C4) not concentric with the optical centre (O) of the optical part 10.

4. Intraocular lens according to any one of Claims 1 to 3, **characterised in that**, for each loop, the area of the section of the shortest leg (21) is greater than the area of the section of the longest leg (22).

5. Intraocular lens according to Claim 4, **characterised in that** the cross-sections of the legs (21, 22) have the shape of a rectangle with rounded corners.

6. Intraocular lens according to any one of Claims 1 to 5, **characterised in that** the junction (213) between each leg (21, 22) of each loop (P_{1.1}, P_{1.2}, P_{2.1}, P_{2.2}) has an asymmetric longitudinal profile.

7. Intraocular lens according to any one of Claims 1 to 6, **characterised in that** the junction (213) between each leg (21, 22) of each loop (P_{1.1}, P_{1.2}, P_{2.1}, P_{2.2}) is comprised within a plane forming a predetermined angle with the plane of the optical part (10).

8. Intraocular lens according to any one of Claims 1 to 7, **characterised in that** the rear face (2131) of the junction is comprised within a plane perpendicular to the optical axis and the front face (2132) of the junction (213) is comprised within a plane forming an acute angle with the optical axis such that backwards projection of the optical part is favoured.

9. Intraocular lens according to any one of Claims 1 to 8, **characterised in that** the intraocular lens is a single-piece lens and is made from a flexible material.

10. Intraocular lens according to one of Claims 1 to 8, **characterised in that** the intraocular lens is of the rigid type, the optical part being rigid and the loops (P_{1.1}, P_{1.2}, P_{2.1}, P_{2.2}) being flexible and added to or machined on the periphery of the optical part.
